# EUROPÄISCHE PATENTANMELDUNG

(11) **EP 0 836 868 A2**
(43) Veröffentlichungstag der Anmeldung: **22.04.1998**
(21) Anmeldenummer: 97116389.4
(22) Anmeldetag: 19.09.1997
(51) Int. Cl.: A61N 1/40

(54) **Hochfrequenzchirurgiegerät und Verfahren zu dessen Betrieb**

(30) Priorität: 18.10.1996 DE 19643127
(71) Anmelder: Gebr. Berchtold GmbH & Co., 78532 Tuttlingen (DE)
(72) Erfinder: Hill, Wolfram, 79102 Freiburg (DE); Dornhof, Konstantin, 78194 Immendingen-Zimmern (DE)
(74) Vertreter: Schmidt, Christian

(57) **Zusammenfassung**

Die Erfindung sieht ein Hochfrequenzchirurgiegerät mit einem Hochfrequenzgenerator (11) mit mindestens einem Aktivelektroden(12)-Ausgang (13) und wenigstens einem Neutralelektroden(14)-Ausgang (15) vor, an den wenigstens eine Teil-Neutralelektrode (14a) eines Neutralelektrodenpaares (14a; 14b) anschließbar ist, deren Einzelelektroden (14a; 14b) an eine Hilfsspannung mit deutlich niedrigerer Frequenz als die Hochfrequenz angeschlossen sind, wobei eine Überwachungsschaltung aus der Hilfsspannung und dem zwischen den Teil-Neutralelektroden (14a; 14b) fließenden Hilfsstrom eine für die Impedanz zwischen den beiden Teil-Neutralelektroden (14a; 14b) repräsentatives Impedanzsignal (19) erzeugt und bei Überschreiten einer ersten festen oberen Alarmgrenze (17) und/oder einer zweiten, an den aktuellen Wert des Impedanzsignals anpaßbaren, oberen Alarmgrenze (20) für das Impedanzsignal (19) ein Hochfrequenzgenerator-Blockierungssignal (22) und/oder ein Alarmsignal (23) abgibt. Erfindungsgemäß ist vorgesehen, daß zur Anpassung der zweiten Alarmgrenze (20) eine SET-Taste (24) vorgesehen ist, bei deren Drücken der automatische Anpassungsvorgang aktiviert wird.

## Beschreibung

Die Erfindung betrifft ein Verfahren zum Betrieb eines Hochfrequenzchirurgiegerätes nach dem Oberbegriff des Patentanspruches 1 und ein Hochfrequenzchirurgiegerät nach dem Oberbegriff des Patentanspruchs 9.

Bei der Anwendung von Hochfrequenzchirurgiegeräten wird meist der monopolare Betriebszustand gewählt, d.h., daß der Hochfrequenzstrom über eine Aktivelektrode dem Gewebe zugeführt und über eine großflächige Neutralelektrode, die an geeigneter Stelle am Körper eines Patienten befestigt ist, abgeführt wird. Früher war diese Neutralelektrode im allgemeinen als ein einheitliches Gebilde ausgeführt, die in allen Bereichen zumindest im wesentlichen auf gleichem Potential liegt. Da bei nicht korrektem Anliegen der Neutralelektrode an die Haut des Patienten Hautverbrennungen auftreten oder auch Nebenströme kapazitiv über die Bedienungsperson abfließen können, ist es besonders wichtig, die korrekte Anlage der Elektrode zu überprüfen.

Ansätze, dieses Problem mit einer einflächigen Elektrode zu lösen, waren nicht befriedigend. Daher wurden Elektroden geschaffen, die mit zwei oder mehr elektrisch gegeneinander isolierten leitenden Flächen ausgerüstet waren und über die dann zusätzlich zum Hochfrequenzstrom Meßströme mit geringerer Frequenz als der Hochfrequenz (die auch Gleichströme einschließen) geschickt werden konnten. Diese Meßströme wurden benutzt, um bei Überschreitung einer vorgegebenen Schwelle einen Alarm auszulösen oder den Hochfrequenzgenerator von den Elektrode zu trennen. Solche Schaltungen sind z.B. in der EP 0 390 937 A1, der DE-OS 28 49 422 oder der DE-OS 35 44 443 beschrieben. Bei diesen bekannten Lösungen gibt es eine fest eingestellte Grenze, bei deren Erreichen bzw. Überschreiten die Übergangsimpedanz der Neutralelektrode als nicht mehr akzeptabel anzusehen ist und damit ein akustischer Alarm ausgelöst wird. Die Überwachungsschaltung wird durch Einstecken der Neutralelektrode aktiviert. Ein Nachteil dieser bekannten Schaltungen besteht darin, daß die Alarmgrenze relativ weit von den tatsächlichen Betriebswerten der Übergangsimpedanz entfernt festgelegt werden muß. Zusätzlich ist eine große und durchaus zulässige Varianz der üblicherweise vorkommenden Übergangswiderstände auch noch durch unterschiedliche Bauformen der Elektroden gegeben.

Es ist auch schon eine Schaltung bekannt (US-PS 48 48 335), bei der durch Drücken einer SET-Taste die momentan vorhandene Impedanz zwischen Neutralelektrode und Gewebe festgestellt und danach die Alarmgrenze eingestellt wird. Auf diese Weise können Unterschiede der Hautbeschaffenheiten verschiedener Patienten sowie Unterschiede in der Bauart der Neutralelektroden bei Festlegung der Alarmgrenzen berücksichtigt werden. Nachteilig an dieser Schaltung ist es jedoch, daß bei der üblichen Verwendung von geteilten Neutralelektroden mit Klebeschicht beim Drücken der SET-Taste unmittelbar nach dem Anliegen der Neutralelektrode zunächst ein zu hoher Widerstand festgestellt und zur Festlegung der Alarmgrenze verwendet wird, während der Kleber erst allmählich in die Hautporen eindringt und erst anschließend der optimale Betriebs-Übergangswiderstand vorliegt, der dann jedoch die Lage der Alarmgrenze nicht mehr beeinflußt.

Eine weitere vorbekannte Schaltung (DE 32 39 640 C2) überwacht den Übergangswiderstand zwischen den beiden Teilelektroden vollautomatisch und verändert die Alarmgrenzen bei langsamen Veränderungen des Übergangswiderstandes selbständig nach oben und unten. Nachteilig an dieser Schaltung ist, daß bei langsamen Veränderungen der Übergangsimpedanz, wie sie auch bei einer langsamen unerwünschten Lösung der Neutralelektrode vom Patienten vorkommen, die Alarmgrenzen unbemerkt vom Operateur auf unempfindlichere Werte gesetzt werden, so daß eine Alarmierung erst bei einer ebenfalls vorgesehenen festen Notalarmschwelle erfolgt, die von den Betriebsimpedanzen ebensoweit entfernt ist wie bei den eingangs erwähnten vorbekannten Schaltungen mit fest eingestellten Alarmgrenzen.

Das Ziel der vorliegenden Erfindung besteht darin, ein Verfahren zum Betrieb eines Hochfrequenzchirurgiegerätes und ein Hochfrequenzchirurgiegerät der eingangs genannten Gattung zu schaffen, bei denen die variable Alarmgrenze durch Berücksichtigung unterschiedlicher Elektrodenarten und -formen sowie unterschiedlicher Hautwiderstände des Patienten optimal an eine gewünschte Betriebsimpedanz anpaßbar ist, ohne daß ein langsames Lösen der Neutralelektrode vom Körper des Patienten erst durch die feste obere Alarmgrenze, d.h. eine Notalarmschwelle bemerkt wird.

Zur Lösung dieser Aufgabe sind die Merkmale der kennzeichnenden Teile der Ansprüche 1 und 9 vorgesehen. Die Aufgabenlösung wird weiter gefördert durch die Merkmale der Patentansprüche 2 bis 7 bzw. 9 bis 15.

Erfindungsgemäß wird also beim Drücken einer SET-Taste kein Momentanwert festgehalten, sondern es wird eine Impedanz-Überwachungsschaltung aktiviert, die beim Absinken der Impedanz zwischen dem Neutralelektrodenpaar und der Haut im Sinne eines besseren Kontaktes die variable zweite Alarmgrenze schärfer macht, d.h. zu niedrigeren Impedanzen hin verschiebt.

Nimmt die Impedanz zwischen den Teilelektroden z.B. aufgrund einer teilweisen Lösung von der Haut des Patienten dagegen zu, folgt erfindungsgemäß die variable zweite Alarmgrenze dieser Veränderung nicht, sondern es kommt in diesem Fall zu einem Alarm, d.h. zu einer Blockierung des Hochfrequenzgenerators (Abschaltung von den Elektrodenausgängen) und/oder zur Abgabe eines optischen oder akustischen Alarmsignals. Der Benutzer muß dann feststellen, ob die Widerstandserhöhung noch auf normale Veränderungen an der Haut des Patienten oder durch ein unerwünschtes teilweises Lösen der Neutralelektrode vom Patienten hervorgerufen ist. Wird eine noch ausreichend gute Anlage der Neutralelektrode festgestellt oder ist eine teilweise gelöste Neutralelektrode erneut einwandfrei am Patienten befestigt worden, betätigt die Bedienungsperson die SET-Taste erneut, wodurch die zweite variable Alarmgrenze nunmehr an den als einwandfrei befundenen Impedanzwert zwischen den beiden Teilelektroden neu angepaßt wird.

Aufgrund der erfindungsgemäßen Anordnung wird also die Bedienungsperson beim Ansteigen der aktuellen Impedanz durch eine Blockierung des Hochfrequenzgenerators und/oder einen Alarm gezwungen, die Neutralelektrode in Augenschein zu nehmen und zu beurteilen, ob die Impedanzerhöhung auf eine im normalen Bereich liegende Erhöhung der Impedanz der Haut oder durch einen Fehler bedingt ist. Dies ist ein wichtiger Sicherheitsaspekt bei der klinischen Anwendung. Wird eine einwandfreie Anlage der Neutralelektrode festgestellt, so daß eine im normalen Bereich liegende Hautwiderstandserhöhung vorliegt, kann der Bediener durch Betätigen der SET-Taste die variable Alarmgrenze entsprechend höhersetzen. Ein wichtiges Merkmal der Erfindung ist es also, daß ohne bewußtes Eingreifen der Bedienungsperson die variable zweite Alarmgrenze nicht unempfindlicher gemacht werden kann. Das Empfindlicherwerden der zweiten Alarmgrenze erfolgt dagegen erfindungsgemäß vollautomatisch ohne Eingreifen der Bedienungsperson.

Nach den Ansprüchen 8 und 16 ist erfindungsgemäß auch eine Kurzschlußerkennung vorgesehen, welche bei Verwendung einflächiger bzw. einpoliger Neutralelektroden ständig ein entsprechendes Signal abgibt, jedoch auch dann anspricht, wenn bei Verwendung von zwei Teil-Neutralelektroden zwischen diesen ein Kurzschluß eintritt. Die Kurzschlußerkennung kann z.B. durch Beobachtung der Wellenform der verwendeten Hilfsspannung erfolgen, welche sich im Kurzschlußfall in charakteristischer Weise verändert, was für die Bildung eines Kurzschlußsignals genutzt werden kann.

Praktische Ausführungsformen des Erfindungsgegenstandes entnimmt man den Patentansprüchen 17 bis 22.

Die Erfindung wird im folgenden beispielsweise anhand der Zeichnung beschrieben; in dieser zeigt:
- Figur 1: ein schematisches Blockschaltbild eines erfindungsgemäßen Hochfrequenzchirurgiegerätes und
- Figur 2: ein Diagramm, welches die Abhängigkeit des von der Impedanz zwischen den beiden Teilelektroden abhängigen Impedanzsignals und der beiden erfindungsgemäßen Alarmgrenzen von der Zeit wiedergibt.

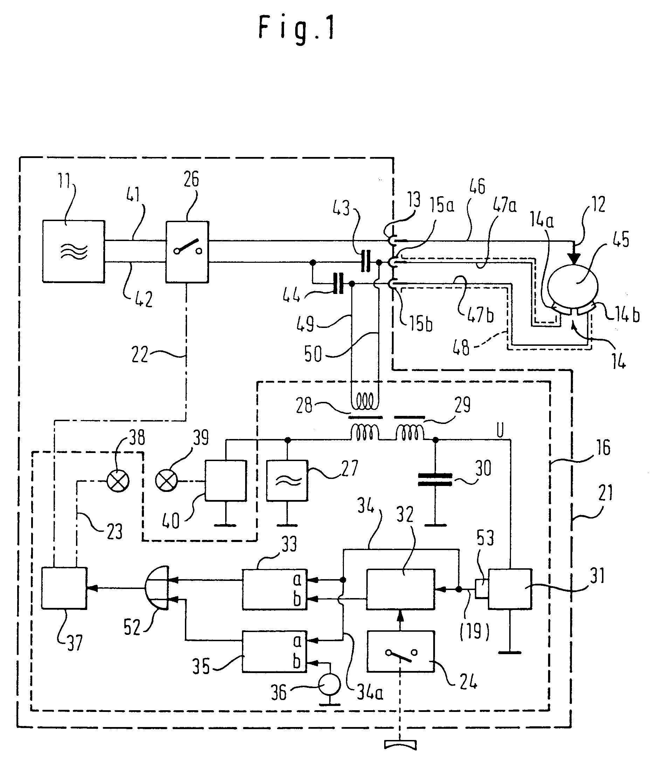

Nach Figur 1 enthält ein erfindungsgemäßes Hochfrequenzchirurgiegerät 21 einen Hochfrequenzgenerator 11, dessen Ausgangsleitungen 41, 42 über eine Schaltvorrichtung 26 an einen Aktivelektrodenausgang 13 und über zwei parallelgeschaltete Trennkondensatoren 43 bzw. 44 an einen Teil-Neutralelektrodenausgang 15a und einen weiteren Teil-Neutralelektrodenausgang 15b angelegt sind. Auch in der Ausgangsleitung 41 zwischen dem Hochfrequenzgenerator 11 und dem Aktivelektroden-Ausgang 13 kann gegebenenfalls ein Trennkondensator oder eine sonstige galvanische Trennung vorgesehen sein.

An den Aktivelektroden-Ausgang 13 ist über eine Verbindungsleitung 46 eine nur schematisch angedeutete Aktivelektrode 12 angeschlossen, die vom Chirurgen mit dem Körper eines Patienten 45 in elektrischen Kontakt gebracht werden kann.

An die Teil-Neutralelektrodenausgänge 15a, 15b sind zu einem gestrichelt angedeuteten Kabel 48 zusammengefaßte Verbindungsleitungen 47a bzw. 47b angeschlossen, die zu elektrisch voneinander isolierten Teil-Neutralelektroden 14a bzw. 14b führen, welche zusammen eine an geeigneter Stelle an den Patienten 45 angelegte und mit dessen Haut in elektrischem Kontakt bringbare Neutralelektrode 14 bilden.

Während die Neutralelektrode 14 einmal an geeigneter Stelle des Patienten 45 fest, jedoch lösbar angebracht wird, wird die Aktivelektrode 12 vom Chirurgen nach Bedarf mit dem zu behandelnden Gewebe in Verbindung gebracht. Im allgemeinen befindet sich an dem die Aktivelektrode 12 enthaltenden Instrument ein vom Chirurgen betätigbarer Schalter, mit dem die Hochfrequenz an den Elektroden 12, 14 aktivierbar ist. Dies kann auch mittels eines nicht dargestellten Fußschalters geschehen.

Für die Überwachung einer einwandfreien Funktion der Neutralelektrode 14 ist erfindungsgemäß eine Überwachungsschaltung 16 wie folgt vorgesehen.

Zwischen den beiden parallel an die Ausgangsleitung 42 angeschlossenen Trennkondensatoren 43, 44 und den Teil-Neutralelektrodenausgängen 15a bzw. 15b zweigen Leitungen 49, 50 ab, die zu der Ausgangswicklung eines Übertragers 28 führen, dessen Primärwicklung einseitig an eine Niederfrequenz-Hilfsspannungsquelle 27 angeschlossen ist, die beispielsweise einen Innenwiderstand von 1 kΩ aufweist und eine Hilfsspannung von etwa 10 V mit einer deutlich niedrigeren Frequenz als die der vom Hochfrequenzgenerator 11 abgegebenen Spannung aufweist. Die Frequenz des Hochfrequenzgenerators 11 kann beispielsweise zwischen 300 kHz und 600 kHz liegen, während die Frequenz der Hilfsspannung z.B. 15 kHz betragen.

An die von der Niederfrequenz-Hilfsspannungsquelle 27 abgewandte Klemme der Primärwicklung des Übertragers 28 ist eine Induktivität 29 angeschlossen, der eine Kapazität 30 folgt, die mit dem vorzugsweise geerdeten anderen Pol der Hilfsspannungsquelle 27 verbunden ist. Die Induktivität 29 und die Kapazität 30 bilden einen Spannungsteiler.

Auf diese Weise wird ein Stromkreis gebildet, dessen Strom in die Primärwicklung des Übertragers 28 eingekoppelt wird und zu den Teil-Neutralelektroden 14a, 14b fließt. Je geringer die Impedanz zwischen den Teil-Neutralelektroden 14a, 14b ist, um so mehr Strom fließt und um so höher wird die Spannung U an der Verbindungsstelle zwischen der Induktivität 29 und der Kapazität 30. Die Spannung U stellt also ein Maß für die Impedanz zwischen den Teil-Neutralelektroden 14a, 14b dar, und zwar in dem Sinne, daß, je höher die Impedanz zwischen den Teil-Neutralelektroden 14a, 14b ist, um so niedriger die Spannung U wird.

Die Verbindungsstelle zwischen der Induktivität 29 und der Kapazität 30 ist an ein Spannungsmeßgerät 31 angeschlossen, an dessen Ausgang ein Spannungssignal U anliegt, dessen Invertierung ein für die Impedanz Z zwischen den Teil-Neutralelektroden 14a, 14b repräsentatives Impedanzsignal darstellt. Obwohl das Spannungssignal U unmittelbar ausgewertet werden kann und vorzugsweise auch wird, ist in Figur 1 zum besseren Verständnis der Funktion eine Invertierungsstufe 53, die den Verlauf der Spannung U umkehrt, als an das Spannungsmeßgerät 31 angelegt angenommen. Am Ausgang der Invertierungsstufe 53 liegt somit ein für die Impedanz zwischen den Teil-Neutralelektorden 14a, 14b repräsentatives Impedanzsignal 19 vor, welches sowohl an den Eingang eines adaptiven Speichers 32 als auch über Leitungen 34, 34a an die Eingänge a eines ersten und zweiten Komparators 33 bzw. 35 angelegt ist, deren Ausgänge einem ODER-Gatter 52 zugeführt sind. Der Ausgang des adaptiven Speichers 32 ist an den zweiten Eingang b des ersten Komparators 33 angelegt.

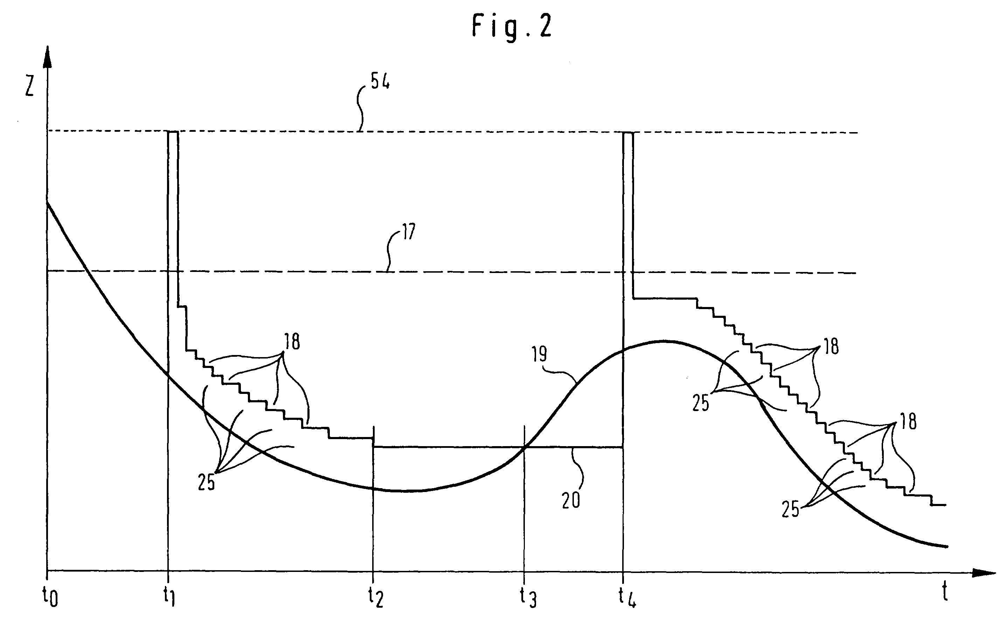

Der adaptive Speicher 32 ist so ausgebildet, daß er nach Drücken der SET-Taste 24 zu einem Zeitpunkt t₁ gemäß Figur 2 einen Alarmgrenzwert 20 speichert, der um einen vorbestimmten Abstand 25 oberhalb des aktuellen Impedanzsignals 19 liegt. Am Ausgang des Speichers 32 erscheint also ein entsprechendes Alarmgrenzsignal. Sobald zum Abstand 25 ein vorbestimmtes Inkrement 18 hinzukommt, wird eine um dieses Inkrement 18 verkleinerte Alarmgrenze gespeichert. Diese sukzessive Speicherung von stufenartig abnehmenden Alarmgrenzen geht so lange vor sich, bis nach Herstellung eines Abstandes 25 zwischen dem aktuellen Wert des Impedanzsignals 19 und der zuvor gespeicherten Alarmgrenze 20 der Abstand nicht mehr kleiner wird und damit die Alarmgrenze erhalten bleibt.

Der Ausgang des ersten Komparators 33 liefert an den einen Eingang des ODER-Gatters 52 ein 0-Signal, wenn das Signal am Eingang a kleiner als am Eingang b ist. Ein L-Signal wird vom Komparator 33 an das ODER-Gatter 52 geliefert, wenn das Signal am Eingang a größer als am Eingang b wird.

An den zweiten Eingang des ODER-Gatters 52 ist der Ausgang des zweiten Komparators 35 angelegt, der die feste obere Sicherheits-Alarmgrenze 17 dadurch bestimmt, daß an seinem einen Eingang a über die Leitungen 34, 34a das Impedanzsignal 19 und an dem zweiten Eingang b eine feste Referenzspannungsquelle 36 angelegt ist. Die Referenzspannung der Referenzspannungsquelle 36 wird werkseitig so eingestellt, daß die beiden Eingangssignale bei a und b gleich werden, wenn über die Leitungen 34, 34a ein der oberen Alarmgrenze 17 entsprechendes Impedanzsignal angelegt wird.

Ist das Impedanzsignal am Eingang a des zweiten Komparators 35 kleiner als das Referenzsignal am Eingang b, erscheint am Ausgang des Komparators 35 ein 0-Signal. Wird das Signal am Eingang a größer als das die Alarmgrenze 17 bestimmende Eingangssignal am Eingang b, erscheint am Ausgang des Komparators 35 ein L-Signal.

Der Ausgang des ODER-Gatters 52 liegt an einer Alarmschaltung 37 an, die über eine Hochfrequenzblockierungs-Signalleitung 22 an die Schaltvorrichtung 26 und über eine Alarmsignalleitung 23 an einen z.B. durch eine LED-Diode gebildeten Alarmsignalgeber 38 angeschlossen ist.

Bei Erscheinen eines L-Signals an einem oder beiden Eingängen des ODER-Gatters 52 wird durch die Alarmschaltung 37 ein Schaltvorgang ausgelöst, der durch Öffnen der Schalter in der Schaltvorrichtung 26 den Hochfrequenzgenerator 11 von den Elektrodenausgängen 13, 15a, 15b trennt und/oder den Alarmsignalgeber 38 zum Ansprechen bringt.

Aufgrund der ODER-Verknüpfung der Ausgangssignale der Komparatoren 33, 35 ist gewährleistet, daß ein Alarm bei Überschreiten der festen Alarmgrenze 17 und/oder beim Überschreiten der Alarmgrenze 20 ausgelöst wird.

Durch Drücken der SET-Taste 24 wird der Anpaßvorgang erneut gestartet, worauf die Alarmgrenze auf einen neuen, insbesondere höheren Werte festgelegt wird, während die feste obere Alarmgrenze 17 eine Sicherheitsgrenze darstellt, die auch durch Einfluß des Bedieners nicht überschritten werden kann. Es kann lediglich durch geeignetes Anlegen der Neutralelektrode 14 erreicht werden, daß das aktuelle Impedanzsignal unterhalb der festen Alarmgrenze 17 liegt.

Der Schaltkreis weist auch eine Kurzschlußerkennungsschaltung 40 auf, die an die Niederfrequenz-Hilfsspannungsquelle 27 angeschlossen ist und einen ebenfalls als LED-Diode ausgebildeten weiteren Alarmsignalgeber 39 beaufschlagt.

Weitere Einzelheiten der beschriebenen Schaltung ergeben sich aus der folgenden Funktionsbeschreibung anhand von Figur 2.

In Figur 2 ist ein typischer Verlauf eines Impedanzsignals 19 wiedergegeben, wie er sich nach dem Anlegen einer Neutralelektrode 14 an einen Patienten 45 einstellt. Die zunächst beim Anlegen der Neutralelektrode 14 zum Zeitpunkt t₀ relativ hohe und oberhalb einer ersten festen Alarmgrenze 17 liegende aktuelle Impedanz fällt nach einigen Sekunden auf Werte unterhalb der durch den Komparator 35 bestimmten festen Alarmgrenze 17 ab und erreicht zu einem Zeitpunkt t₂ annähernd ein erstes Minimum.

Es sei nun angenommen, daß zu einem Zeitpunkt t₁ nach dem Anlegen der Neutralelektrode 14 die SET-Taste 24 gedrückt wird. Hierdurch wird ausgehend von einem oberhalb der festen Alarmgrenze 17 liegenden Reset-Pegel 54 der Anpaßvorgang gestartet und die zweite, variable Alarmgrenze 20 so festgelegt, daß sie zunächst um einen vorbestimmten Abstand 25 oberhalb des aktuellen Impedanzsignals 19 liegt. Diese Alarmgrenze 20 wird zunächst so lange im adaptiven Speicher 32 festgehalten, bis zum Abstand 25 zwischen der Alarmgrenze 20 und dem aktuellen Impedanzsignal 19 eine vorbestimmte Hysterese 18 hinzugekommen ist, worauf im Speicher 32 nunmehr ein etwas niedrigerer Impedanzwert, der wieder den Abstand 25 vom aktuellen Impedanzsignal 19 aufweist, gespeichert und erneut so lange festgehalten wird, bis zum Abstand 25 wieder das Inkrement 18 hinzugekommen ist.

Dieses schrittweise Absenken der Alarmgrenze 20 im adaptiven Speicher 32 wird so lange fortgesetzt, bis das aktuelle Impedanzsignal 19 zum Zeitpunkt t₂ das Minimum oder einen konstant bleibenden Wert erreicht. Von nun an bleibt die Alarmgrenze 20 zunächst konstant. Überschreitet zum Zeitpunkt t₃ das aktuelle Impedanzsignal 19 die Alarmgrenze 20, wird ein Alarm ausgelöst, d.h. daß der Hochfrequenzgenerator 11 mittels der Schaltvorrichtung 26 von den Ausgangsklemmen 13, 15a, 15b abgeschaltet wird und/oder der Alarmgeber 38 anspricht. Dieser Alarm bleibt solange aufrechterhalten, bis die SET-Taste erneut gedrückt wird.

Wenn der Bediener das Abschalten der Hochfrequenz festgestellt und/oder das Alarmsignal wahrgenommen hat, kann er die Neutralelektrode 14 untersuchen und für den Fall, daß er einen einwandfreien Sitz am Körper des Patienten 45 feststellt zum Zeitpunkt t₄ die SET-Taste 24 erneut drücken, worauf in der in Figur 2 dargestellten Weise wieder ausgehend vom Reset-Pegel 54 und ganz analog wie zum Zeitpunkt t₁ eine neue Adaption der variablen zweiten Alarmgrenze 20 an den aktuellen Wert des Impedanzsignals 19 erfolgt. Die Alarmgrenze 20 wird also zunächst zu noch unter der oberen Grenze 17 liegenden, höheren Impedanzwerten verschoben, paßt sich dann aber beim erneuten Absinken des Impedanzsignals 19 vollautomatisch an das aktuelle Impedanzsignal 19 an, so daß die Alarmgrenze 20 wieder schärfer gemacht wird.

Sollte die aktuelle Impedanz 19 die erste obere Alarmgrenze 17 überschreiten und der Bediener die SET-Taste betätigen, so verhindert der zweite Komparator 35 aufgrund einer der ersten Alarmgrenze 17 entsprechend gewählten Referenzspannung der Referenzspannungsquelle 36, eine erneute Deblockierung des Hochfrequenzgenerators 11 bzw. ein Erlöschen des Alarmsignalgebers 38. Eine erneute Inbetriebsetzung ist also erst dann möglich, wenn das aktuelle Impedanzsignal 19 die erste Alarmgrenze 17 wieder unterschritten hat. Dies kann z.B. vom Bediener durch eine bessere Befestigung der Neutralelektorde 14 herbeigeführt werden.

In Figur 2 ist an der Ordinate auch ein Pfeil für den Verlauf der Spannung U am Ausgang des Spannungsmeßgerätes 31 angebracht. Wenn die Grenzen 17, 20 und der Kurvenverlauf 19 der Spannung U zugeordnet werden, wären der adaptive Speicher 32 sowie die Komparatoren 33, 35 so abzuwandeln, daß die Größenverhältnisse der einzelnen Werte gerade umgekehrt als vorstehend beschrieben gewählt werden, nachdem ja der Verlauf von Spannung U und Impedanz R gerade gegenläufig ist.

Wenn zwischen den Teil-Neutralelektroden 14a, 14b ein Kurzschluß entsteht oder wenn an die beiden Elektrodenausgänge 15a, 15b eine einflächige bzw. einpolige Neutralelektrode 14 angeschlossen wird, erkennt die Kurzschlußerkennungsschaltung 40 einen Kurzschluß, der durch Ansprechen des Alarmsignalgebers 39 dem Bediener angezeigt wird.

### Bezugszeichenliste

- 11: Hochfrequenzgenerator
- 12: Aktivelektrode
- 13: Aktivelektroden-Ausgang
- 14: Neutralelektrode
- 14a: Teil-Neutralelektrode
- 14b: Teil-Neutralelektrode
- 15a: Teil-Neutralelektrodenausgang
- 15b: Teil-Neutralelektrodenausgang
- 16: Überwachungsschaltung
- 17: feste obere Alarmgrenze
- 18: Inkrement
- 19: Impedanzsignal
- 20: zweite variable Alarmgrenze
- 21: Hochfrequenzchirurgiegerät
- 22: Hochfrequenzblockierungs-Signalleitung
- 23: Alarmsignalleitung
- 24: SET-Taste
- 25: vorbestimmter Abstand
- 26: Schaltvorrichtung
- 27: Niederfrequenz-Hilfsspannungsquelle
- 28: Übertrager
- 29: Induktivität
- 30: Kapazität
- 31: Spannungsmeßgerät
- 32: adaptiver Speicher
- 33: erster Komparator
- 34: Leitung
- 35: zweiter Komparator
- 36: Referenzspannungsquelle
- 37: Alarmschaltung
- 38: Alarmsignalgeber
- 39: Alarmsignalgeber
- 40: Kurzschlußerkennungsschaltung
- 41: Ausgangsleitung
- 42: Ausgangsleitung
- 43: Trennkondensator
- 44: Trennkondensator
- 45: Patient
- 46: Verbindungsleitung
- 47a: Verbindungsleitung
- 47b: Verbindungsleitung
- 48: Kabel
- 49: Leitung
- 50: Leitung
- 51: verstellbare Referenzspannungsquelle
- 52: ODER-Gatter
- 53: Invertierungsstufe
- 54: Reset-Pegel

## Patentansprüche

1. Verfahren zum Betrieb eines Hochfrequenzchirurgiegerätes (21) mit einem Hochfrequenzgenerator (11) mit mindestens einem Aktivelektroden(12)-Ausgang (13) und wenigstens einem Neutralelektroden(14)-Ausgang (15), an den wenigstens eine Teil-Neutralelektrode (14a) eines Neutralelektrodenpaares (14a; 14b) anschließbar ist, deren Einzelelektroden (14a; 14b) an eine Hilfsspannung mit deutlich niedrigerer Frequenz als der Hochfrequenz angeschlossen sind, wobei eine Überwachungsschaltung aus der Hilfsspannung und dem zwischen den Teil-Neutralelektroden (14a; 14b) fließenden Hilfsstrom eine für die Impedanz zwischen den beiden Teil-Neutralelektroden (14a; 14b) repräsentatives Impedanzsignal (19) erzeugt und bei Überschreiten einer ersten festen oberen Alarmgrenze (17) und/oder einer niedrigeren zweiten, an den aktuellen Wert des Impedanzsignals anpaßbaren, oberen Alarmgrenze (20) für das Impedanzsignal (19) ein Hochfrequenzgenerator-Blockierungssignal (22) und/oder ein Alarmsignal (23) abgibt,
dadurch **gekennzeichnet,**
daß die Anpassung der zweiten Alarmgrenze (20) durch Drücken einer SET-Taste (24) vorgenommen wird.

2. Verfahren nach Anspruch 1,
dadurch **gekennzeichnet,**
daß die Anpassung darin besteht, daß die zweite Alarmgrenze (20) zunächst auf einen Wert oberhalb des aktuellen Impedanzsignals (19) und vorzugsweise bis zur oder oberhalb der festen ersten Alarmgrenze (17) bis maximal zu einem Reset-Pegel (54) verstellt wird und anschließend vorzugsweise stufenweise bis auf einen vorbestimmten Abstand (25) dem aktuellen Wert des Impedanzsignals (19) angenähert wird.

3. Verfahren nach Anspruch 2,
dadurch **gekennzeichnet,**
daß die zweite Alarmgrenze (20) nach erfolgter Annäherung dem aktuellen Wert des Impedanzsignals (19) nachgeführt wird.

4. Verfahren nach Anspruch 3,
dadurch **gekennzeichnet,**
daß die Nachführung der zweiten Alarmgrenze (20) nur bei niedriger werdendem Impedanzsignal (19) vorgenommen wird.

5. Verfahren nach Anspruch 4,
dadurch **gekennzeichnet,**
daß die zweite Alarmgrenze (20) dem Impedanzsignal kontinuierlich nachgeführt wird oder nach der erfolgten Annäherung festgehalten wird, bis der aktuelle Wert des Impedanzsignals sich von der festgehaltenen zweiten Alarmgrenze (20) um ein bestimmtes Inkrement (18) entfernt hat, worauf die zweite Alarmgrenze (20) bis zur Wiederherstellung des vorbestimmten Abstandes (25) nachgeführt wird.

6. Verfahren nach einem der vorhergehenden Ansprüche,
dadurch **gekennzeichnet,**
daß bei Überschreitung der ersten oder zweiten Alarmgrenze (17, 20) durch den aktuellen Wert des Impedanzsignals (19) eine Blockierung (22) des Hochfrequenzgenerators (11) und/oder die Auslösung eines Alarmsignals (23) erfolgt.

7. Verfahren nach Anspruch 6,
dadurch **gekennzeichnet,**
daß die Blockierung (22) des Hochfrequenzgenerators (11) und/oder das Alarmsignal (23) erst durch erneutes Betätigen der SET-Taste (24) und das darauf erfolgende Anpassen der zweiten Alarmgrenze (20) an den aktuellen Wert des Impedanzsignals (19) aufgehoben wird, sofern das Impedanzsignal (19) kleiner als die erste feste Alarmgrenze (17) wird.

8. Verfahren nach einem der vorhergehenden Ansprüche,
dadurch **gekennzeichnet,**
daß durch die Überwachungsschaltung (16) auch eine Kurzschlußerkennung vorgenommen wird und daß bevorzugt die Kurzschlußerkennung zur Unterscheidung und/oder Anzeige der Verwendung einer einteiligen statt einer mehrteiligen Neutralelektrode (14) dient.

9. Hochfrequenzchirurgiegerät (21) mit einem Hochfrequenzgenerator (11) mit mindestens einem Aktivelektroden(12)-Ausgang (13) und wenigstens einem Neutralelektroden(14)-Ausgang (15), an den wenigstens eine Teil-Neutralelektrode (14a) eines Neutralelektrodenpaares (14a; 14b) anschließbar ist, deren Einzelelektroden (14a; 14b) an eine Hilfsspannung mit deutlich niedrigerer Frequenz als der Hochfrequenz angeschlossen sind, wobei eine Überwachungsschaltung aus der Hilfsspannung und dem zwischen den Teil-Neutralelektroden (14a; 14b) fließenden Hilfsstrom eine für die Impedanz zwischen den beiden Teil-Neutralelektroden (14a; 14b) repräsentatives Impedanzsignal (19) erzeugt und bei Überschreiten einer ersten festen oberen Alarmgrenze (17) und/oder einer zweiten, an den aktuellen Wert des Impedanzsignals anpaßbaren, oberen Alarmgrenze (20) für das Impedanzsignal (19) ein Hochfrequenzgenerator-Blockierungssignal (22) und/oder ein Alarmsignal (23) abgibt, insbesondere zur Ausführung des Verfahrens nach einem der vorhergehenden Ansprüche,
dadurch **gekennzeichnet,**
daß zur Anpassung der zweiten Alarmgrenze (20) eine SET-Taste (24) vorgesehen ist, bei deren Drücken eine automatische Anpassung an das aktuelle Impedanzsignal (19) ausgelöst wird.

10. Vorrichtung nach Anspruch 9,
dadurch **gekennzeichnet,**
daß die Anpassung darin besteht, daß sich die zweite Alarmgrenze (20) zunächst auf einen Wert oberhalb des aktuellen Impedanzsignals (19) und vorzugsweise bis zur oder oberhalb der festen ersten Alarmgrenze (17) bis zu einem Reset-Pegel (54) rückgestellt wird und sich anschließend vorzugsweise stufenweise automatisch bis auf einen vorbestimmten Abstand (25) dem aktuellen Wert des Impedanzsignals (19) annähert.

11. Hochfrequenzchirurgiegerät nach Anspruch 10,
dadurch **gekennzeichnet,**
daß die zweite Alarmgrenze (20) anschließend automatisch dem aktuellen Wert des Impedanzsignals nachgeführt wird.

12. Hochfrequenzchirurgiegerät nach Anspruch 11,
dadurch **gekennzeichnet,**
daß die Nachführung der zweiten Alarmgrenze (20) nur bei niedriger werdendem Impedanzsignal (19) erfolgt.

13. Hochfrequenzchirurgiegerät nach Anspruch 12,
dadurch **gekennzeichnet,**
daß die zweite Alarmgrenze (20) dem Impedanzsignal kontinuierlich nachgeführt wird oder nach der erfolgten Annäherung automatisch festgehalten wird, bis der aktuelle Wert des Impedanzsignals (19) sich von der festgehaltenen zweiten Alarmgrenze (20) um ein bestimmtes Inkrement (18) entfernt hat, worauf die zweite Alarmgrenze (20) automatisch bis zur Wiederherstellung des vorbestimmten Abstandes (25) nachgeführt wird.

14. Hochfrequenzchirurgiegerät nach einem der Ansprüche 9 bis 13,
dadurch **gekennzeichnet,**
daß bei Überschreitung der ersten oder zweiten Alarmgrenze (17, 20) durch den aktuellen Wert des Impedanzsignals (19) ein Blockierungssignal (22) an eine Schaltvorrichtung (26) für den Hochfrequenzgenerator (11) und/oder ein Alarmsignal (23) an einen Alarmsignalgeber (38) abgegeben wird.

15. Hochfrequenzchirurgiegerät nach Anspruch 14,
dadurch **gekennzeichnet,**
daß die Schaltvorrichtung (26) für die Blockierung des Hochfrequenzgenerators (11) und/oder der Alarmsignalgeber (38) erst durch erneutes Betätigen der SET-Taste (24) und das darauffolgende Anpassen der zweiten Alarmgrenze (20) an den aktuellen Wert des Impedanzsignals (29) die Blockierung bzw. Alarmgebung aufhebt, sofern das Impedanzsignal (19) unter die feste obere Alarmgrenze (17) abgesunken ist.

16. Hochfrequenzchirurgiegerät nach einem der Ansprüche 9 bis 15,
dadurch **gekennzeichnet,**
daß in der Überwachungsschaltung (16) auch eine Kurzschlußerkennung (39, 40) vorgesehen ist und daß bevorzugt die Kurzschlußerkennung zur Unterscheidung und/oder Anzeige der Verwendung einer einteiligen statt einer mehrteiligen Neutralelektrode (14) dient.

17. Hochfrequenzchirurgiegerät nach einem der Ansprüche 9 bis 16,
dadurch **gekennzeichnet,**
daß die Überwachungsschaltung (16) eine Niederfrequenz-Hilfsspannungsquelle (27) aufweist, die die Hilfsspannung über einen Übertrager (28) an die Teil-Neutralelektroden (14a; 14b) anlegt.

18. Hochfrequenzchirurgiegerät nach Anspruch 17,
dadurch **gekennzeichnet,**
daß der Übertrager (28) mit einer Induktivität (29) und einer Kapazität (30) in Reihe geschaltet ist und ein Spannungsmeßgerät (31) für die Messung der das inverse Impulssignal (19) darstellenden Spannung an der Verbindungsstelle zwischen der Induktivität (29) und der Kapazität (30) vorgesehen ist.

19. Hochfrequenzchirurgiegerät nach einem der Ansprüche 9 bis 18,
dadurch **gekennzeichnet,**
daß das Spannungssignal oder das daraus gebildete Impedanzsignal (19) an einen adaptiven Speicher (22) angelegt ist, dessen Ausgang an den einen Eingang (b) eines ersten Komparators (33) angelegt ist, dessen anderem Eingang (a) das aktuelle Spannungs- bzw. Impedanzsignal (19) zugeführt ist.

20. Hochfrequenzchirurgiegerät nach einem der Ansprüche 9 bis 19,
dadurch **gekennzeichnet,**
daß ein zweiter Komparator (35) vorgesehen ist, der die erste obere Alarmgrenze (17) festlegt und vorzugsweise an einem Eingang (a) vom Spannungs- bzw. Impedanzsignal (19) und am anderen Eingang (b) von einer festen Referenzspannung (36) beaufschlagt ist.

21. Hochfrequenzchirurgiegerät nach Anspruch 20,
dadurch **gekennzeichnet,**
daß die Ausgänge der Komparatoren (33, 35) an ein ODER-Gatter (52) angeschlossen sind, dem eine Alarmschaltung (37) folgt, an die eine zwischen den Hochfrequenzgenerator (11) und die Elektrodenausgänge (13; 15a, 15b) geschaltete Schaltvorrichtung (26) und/oder ein Alarmsignalgeber (38) angelegt ist.

22. Hochfrequenzchirurgiegerät nach einem der Ansprüche 9 bis 21,
dadurch **gekennzeichnet,**
daß an die Hilfsspannungsquelle (27) eine Kurzschluß-Erkennungsschaltung (40) angelegt ist, die einen Alarmsignalgeber (39) beaufschlagt.
